(19) Europäisches Patentamt
European Patent Office
Office européen des brevets

(11) EP 1 172 079 A1

(12) **DEMANDE DE BREVET EUROPEEN**

(43) Date de publication:
**16.01.2002 Bulletin 2002/03**

(51) Int Cl.[7]: **A61K 7/043**

(21) Numéro de dépôt: **01401581.2**

(22) Date de dépôt: **15.06.2001**

(84) Etats contractants désignés:
**AT BE CH CY DE DK ES FI FR GB GR IE IT LI LU MC NL PT SE TR**
Etats d'extension désignés:
**AL LT LV MK RO SI**

(30) Priorité: **13.07.2000 FR 0009224**

(71) Demandeur: **L'OREAL**
**75008 Paris (FR)**

(72) Inventeurs:
- **Jeanne-Rose, Valérie**
  **75019 Paris (FR)**
- **Quinn, Francis**
  **75005 Paris (FR)**

(74) Mandataire: **Miszputen, Laurent**
**L'OREAL-DPI 6 rue Bertrand Sincholle**
**92585 Clichy Cédex (FR)**

(54) **Utilisation d'un composé métallo-organique pour protéger et/ou renforcer les matières kératiniques, et procédé de traitement**

(57) La présente demande concerne l'utilisation d'une composition comprenant au moins un composé métallo-organique susceptible d'être obtenu par hydrolyse, partielle ou complète, et condensation, partielle ou complète, d'au moins un précurseur métallique, pour protéger et/ou renforcer les matières kératiniques.

EP 1 172 079 A1

## Description

La présente invention a trait à l'utilisation de matériaux obtenus par hydrolyse et condensation notamment d'alcoxydes métalliques, pour le traitement cosmétique des matières kératiniques, notamment des ongles.

Il est bien connu que les ongles présentent, de façon fréquente, des défauts de structure et de consistance, ceux-ci pouvant être d'origines diverses, notamment liées au fonctionnement interne de l'individu, à ses conditions de vie, à son mode d'alimentation, à son âge, à ses états de fatigue ou de surmenage. Ces défauts peuvent également apparaître sous l'effet d'actions qui érodent, à la suite par exemple d'expositions prolongées ou répétées à des agents détergents, à des solvants, à des produits chimiques en particulier ménagers, à des atmosphères chaudes ou froides, humides ou sèches, ou à des expositions aux rayonnements UV. Ces défauts de structure et de consistance ont pour effet de rendre la surface des ongles inesthétique, ce qui peut être source de gêne et de désagréments multiples.

En vue de renforcer les ongles, on a déjà proposé différents types de compositions essentiellement basées sur l'emploi soit d'agents réticulants des protéines destinées à renforcer le réseau kératinique comme par exemple le formol, soit d'agents à fonction essentiellement nutritive tels que par exemple la cystine, le cholestérol, la S-carboxyméthylcystéïne ou encore des extraits de collagène. L'utilisation de tels agents réticulants ou de tels agents à fonction nutritive ne permet pas cependant d'obtenir de bons résultats et présente par ailleurs certains inconvénients. En particulier, les produits à base de formol peuvent provoquer certaines réactions d'allergie. On a également proposé d'utiliser de l'acide silicique colloïdal en tant qu'agent renforçateur desdites matières kératiniques, notamment des ongles, mais également des cheveux et des cils. Ces compositions permettent d'améliorer la qualité des ongles, c'est-à-dire de les rendre moins fragiles, moins cassants ou moins mous. Toutefois, la plupart des compositions existantes nécessitent plusieurs applications avant que l'on puisse observer un effet. En outre, selon la nature de l'agent renforçateur employé, il peut être nécessaire d'éviter le contact avec l'eau, l'effet ne résistant pas à un trempage prolongé des ongles. Ceci peut notamment être dû au fait que les agents renforçateurs selon l'art antérieur n'agissent qu'en surface des matières kératiniques.

Il existe donc toujours le besoin de disposer d'une composition cosmétique qui, par simple application sur les matières kératiniques telles que ongles, cils, sourcils, poils ou cheveux, permet d'en améliorer très rapidement la qualité, notamment de les protéger et/ou de les renforcer.

La présente invention a pour but de proposer une telle composition, qui permet notamment de protéger et/ou renforcer les matières kératiniques, notamment en en améliorant de manière rapide et durable, la rigidité et/ou la cohésion.

Un objet de la présente invention est donc l'utilisation d'une composition comprenant au moins un composé métallo-organique susceptible d'être obtenu par hydrolyse, partielle ou complète, et condensation, partielle ou complète, d'au moins un précurseur métallique choisi parmi ceux décrits ci-après, pour protéger et/ou renforcer les matières kératiniques.

Un autre objet de l'invention est un procédé de traitement des matières kératiniques, notamment pour les protéger et/ou les renforcer, consistant à appliquer sur lesdites matières une composition comprenant au moins un composé métallo-organique susceptible d'être obtenu par hydrolyse, partielle ou complète, et condensation, partielle ou complète, d'au moins un précurseur métallique tel que défini ci-après.

La composition selon l'invention, lorsqu'elle est à appliquer sur l'ongle, peut notamment permettre de diminuer la fragilisation des ongles, notamment des ongles affaiblis, en particulier des ongles striés, fendus, mous, souples, et/ou ayant tendance à se dédoubler.

On a constaté que l'application sur la surface de l'ongle de ladite composition permet d'augmenter la rigidité et la cohésion de l'ongle, et ce dès la première application; par ailleurs, cet effet persiste dans le temps, et est rémanent à l'eau.

L'utilisation d'une composition selon l'invention permet l'obtention d'ongles plus durs et plus résistants, donc moins cassants. Ce renforcement de la kératine de l'ongle permet également d'obtenir des ongles qui ne se dédoublent plus et/ou qui ne se fendillent plus.

Lorsque la composition est destinée à être appliquée sur les cheveux, elle peut permettre de rigidifier les cheveux, notamment les cheveux mous, et d'en améliorer ainsi le coiffage.

L'utilisation selon l'invention trouve donc une application toute particulière pour traiter les ongles fragiles, cassants ou mous, mais également pour les ongles normaux, ou pour les cils, les sourcils et les cheveux.

Sans être tenu par la présente explication, on peut penser que ceci est dû à la formation, au sein de la matière kératinique, d'un réseau tri-dimensionnel généré par le composé métallo-organique, ce qui va se traduire par une augmentation de sa dureté ou rigidité.

Par ailleurs, la composition selon l'invention peut permettre d'apporter d'autres propriétés à la matière kératinique, telles que des effets optiques, des effets de surface, notamment de lissage de la surface et/ou une modification de la mouillabilité de ladite surface.

La composition qui est employée dans le cadre de la présente invention comprend donc, notamment dans un milieu cosmétiquement acceptable, au moins un composé métallo-organique susceptible d'être obtenu par hydrolyse et condensation, partielles et/ou totales, d'au moins un précurseur métallique choisi parmi, seul ou en mélange :

(1) les alcoxydes métalliques répondant à l'une des formules (Ia), (Ib), (Ic) ou (Id) suivantes :

$$M\text{-}(OR_1)_n \quad \text{(I a)}$$

$$R\text{-}M\text{-}(OR_1)_{n-1} \quad \text{(I b)}$$

$$(R_1O)_{n-1}\text{-}M\text{-}R''\text{-}M'\text{-}(OR_1)_{n-1} \quad \text{(I c)}$$

$$RR'\text{-}M\text{-}(OR_1)_{n-2} \quad \text{(I d)}$$

dans lesquelles :

- M et M', indépendamment l'un de l'autre, désignent un atome de métal choisi parmi les métaux de transition des groupes Ib à VIIb, du groupe VIII ou du groupe des lanthanides de la classification périodique, l'aluminium, le silicium, le bore, l'étain et le magnésium, les métaux alcalins et alcalino-terreux;
- n désigne la valence du métal;
- $R_1$, identiques ou différents, désignent un radical hydrocarboné, linéaire ou ramifié, saturé ou insaturé, ayant 1 à 30 atomes de carbone, de préférence 1 à 6 atomes de carbone, éventuellement interrompu ou substitué par 1-20 hétéroatomes choisis parmi O, N, S et/ou P;
- R et R', indépendamment l'un de l'autre, désignent un atome d'hydrogène ou un radical hydrocarboné, linéaire, ramifié ou cyclique, saturé ou insaturé, en C1-30, de préférence C2-20, éventuellement substitué ou interrompu par 1-20 hétéroatomes choisis parmi O, N, S et/ou P; et /ou substitué par un groupement choisi dans la liste ci-après;
  lesdits R et/ou R' pouvant comporter en outre un ou plusieurs groupes cosmétiquement actifs tels que définis ci-après;
- R" désigne -O-, $-NR^2-$, -S- ou un radical divalent hydrocarboné, linéaire, cyclique ou ramifié, saturé ou insaturé, en C1-30, de préférence C2-20, éventuellement substitué par un groupement choisi dans la liste ci-après; et/ou éventuellement interrompu ou substitué par 1-20 hétéroatomes choisis parmi O, N, P et/ou S, pouvant comporter en outre un ou plusieurs groupes cosmétiquement actifs tels que définis ci-après, avec $R^2$ représentant un radical hydrocarboné, linéaire, cyclique ou ramifié, saturé ou insaturé, en C1-30, de préférence C2-20;

(2) les complexes répondant à l'une des formules (IIa), (IIb), (IIc) ou (IId) suivantes :

$$M\text{-}(OR_1)_{n-x}(X)_x \quad \text{(II a)}$$

$$R\text{-}M\ (OR_1)_{n-1-x}(X)_x \quad \text{(II b)}$$

$$(X)_x\ (R_1O)_{n-1-x}\ M\text{-}R''\text{-}M'\text{-}(OR_1)_{n-1-x}\ (X)_x \quad \text{(II c)}$$

$$RR'\text{-}M\text{-}(OR_1)_{n-x-2}\ (X)_x \quad \text{(II d)}$$

dans lesquelles :

- M, M', n, R, R', R" et $R_1$ ont la même signification que ci-dessus;
- X désigne un ligand comportant un atome d'azote, de phosphore, de soufre et/ou d'oxygène, et portant éventuellement un groupe cosmétiquement actif tel que défini ci-après;

- x est le nombre d'atomes pouvant se lier à l'atome central métallique;

(3) les halogénures métalliques répondant à l'une des formules (IIIa), (IIIb), (IIIc) ou (IIId) suivantes :

$$M\text{-}(Z)_n \qquad \text{(III a)}$$

$$R\text{-}M\text{-}(Z)_{n-1} \qquad \text{(III b)}$$

$$(Z)_{n-1}\text{-}M\text{-}R''\text{-}M'\text{-}(Z)_{n-1} \qquad \text{(III c)}$$

$$RR'\text{-}M\text{-}(Z)_{n-2} \qquad \text{(III d)}$$

dans lesquelles :

- M, M', n, R, R' et R" ont la même signification que ci-dessus;
- Z représente, indépendamment l'un de l'autre, un atome d'halogène (chlore, iode, brome ou fluor);

(4) les complexes répondant à l'une des formules (IVa), (IVb), (IVc) ou (IVd) suivantes :

$$M\text{-}(Z)_{n-x}\ (X)_x \qquad \text{(IVa)}$$

$$R\text{-}M\ (Z)_{n-1-x}\ (X)_x \qquad \text{(IVb)}$$

$$(X)_x\ (Z)_{n-1-x}\ M\text{-}R''\text{-}M'\text{-}(Z)_{n-1-x}\ (X)_x \qquad \text{(IVc)}$$

$$RR'\text{-}M\text{-}(Z)_{n-x-2}\ (X)_x \qquad \text{(IVd)}$$

dans lesquelles :

- M, M', n, R, R', R", X, x et Z ont la même signification que ci-dessus.

Selon la présente invention, on entend par ligand un groupe comportant au moins un atome pouvant se lier à l'atome central métallique.

Parmi les substituants susceptibles d'être portés par R, R' et/ou R", on peut citer les atomes d'halogènes (chlore, brome, iode et/ou fluor) et les groupements suivants : $-NR_2$, $-CO-NR_2$, -SR, -R-S-R, $-CO_2R$,-COR, -OH, -N=C=O,$-NR-CO-NR_2$, $-N^+R_3$, $-S^+=C(NR_2)_2$; sulfonate ($-SO_3-R$);

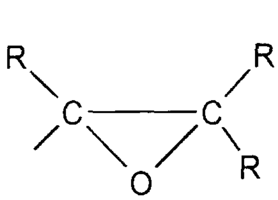
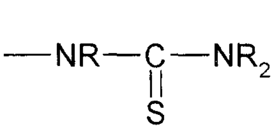
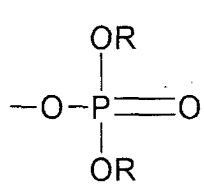
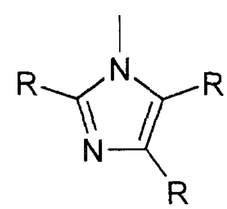

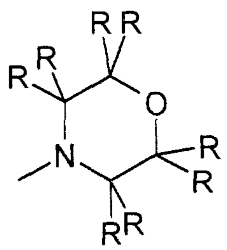
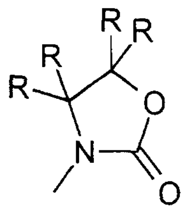
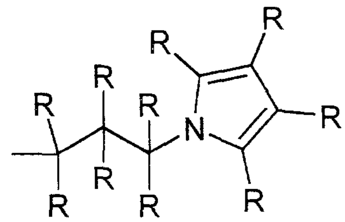

dans lesquels les différents radicaux R, identiques ou différents, désignent un atome d'hydrogène ou un radical hydrocarboné, linéaire, ramifié ou cyclique, saturé ou insaturé, en C1-30, de préférence C2-20.

Parmi les groupes cosmétiquement actifs susceptibles d'être portés par R, R', R" et/ou X, on peut citer un groupe colorant; un groupe photochrome; un groupe filtrant les radiations UV-A et/ou UV-B; un groupe promoteur d'adhésion sur les matières kératiniques tel qu'un groupe amide, uréthanne, urée, hydroxyle, carboxyle, acide aminé ou polypeptide; un groupe facilitant le démaquillage; un groupe bactéricide ou bactériostatique; un groupe chélatant en particulier pouvant complexer de cations multivalents; un hydroxyacide; un groupe anti-chute des cheveux; un groupe antioxydant; un groupe anti-radicaux libres; un groupe porteur de vitamine.

De préférence, le précurseur métallique est choisi par les composés répondant aux formules (Ia), (Ib), (Ic), (Id) et (IIa), et plus particulièrement aux formules (Ia), (Ib) et (IIa).

De préférence, on choisit l'atome de métal M parmi le titane, le zirconium, l'aluminium, le fer, l'étain et le silicium, et plus particulièrement parmi le titane et le silicium.

De préférence, $R_1$ désigne, identique ou différent, un radical hydrocarboné, linéaire ou ramifié, saturé, ayant 1 à 30 atomes de carbone, de préférence 1 à 6 atomes de carbone, et plus particulièrement un radical méthyl, éthyl, propyl, n-butyl, isobutyl ou t-butyl.

De préférence, R et R' désignent, indépendamment l'un de l'autre, un radical hydrocarboné, linéaire ou ramifié, saturé, en C1-20, notamment C1-6; ou un radical hydrocarboné, linéaire ou ramifié, saturé, en C1-20, notamment C1-6, substitué par un ou plusieurs atomes d'halogène (notamment perfluoré), ou par un groupement $-NH_2$, $-CO-NH_2$, -SH, $-CO_2H$,-COR, -OH, -N=C=O,$-NH-CO-NH_2$, $-N^+R_3$ et notamment $-N^+Bu_3$, $-S^+=C(NH_2)_2$; benzènesulfonate;

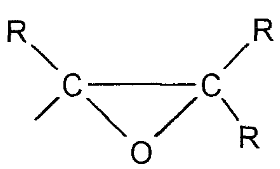
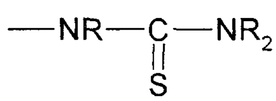
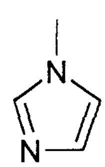
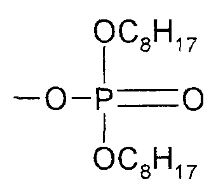

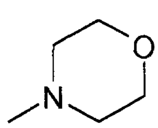
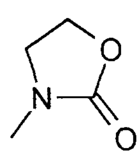
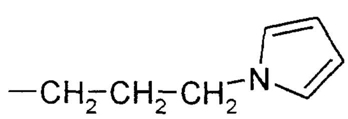

dans lesquels les différents radicaux R, identiques ou différents, désignent un atome d'hydrogène ou un radical hydrocarboné, linéaire, ramifié ou cyclique, saturé ou insaturé, en C1-30, de préférence C2-20.

De préférence, R" désigne -O-, -NH- ou un radical divalent hydrocarboné, linéaire ou ramifié, saturé, en C1-30, de préférence C2-20, éventuellement interrompu par au moins un hétéroatome choisi parmi O, N, P et/ou S.

De préférence, X peut être choisi parmi les acides carboxyliques, les acides sulfoniques, les acides phosphoniques, les acides phosphoriques, les acides sulfuriques, les cétones, les β-dicétones, les esters, les β-cétoesters, les amines, les β-cétoamines, les acides aminés de préférence α- ou β-hydroxylés et leurs dérivés, les α- ou β-hydroxyacides, les éthers et polyéthers, les imines, les amides éventuellement hydroxylées, les composés azoïques, les thiols, les ureidos, les sulfoxydes de thioéther, les sulfones de thioéther, les thioéthers éventuellement cycliques, les di(thioéthers), les monoalcools ou les polyols, la dextrine et ses dérivés, les thiazolidines; les polymères hydrocarbonés comportant éventuellement des hétéroatomes choisis parmi N, O, S et/ou P, susceptibles d'être obtenus par polymérisation radicalaire, par condensation ou par polymérisation 'vivante' contrôlée, ayant un poids moléculaire (en masse) compris entre 90 et 10000, notamment de 100 à 1000, voire de 150 à 500; et leurs dérivés.

On peut notamment citer :

- l'acide salicylique et ses dérivés tels que l'acide (méth)acrylamino-4-salicylique et l'acide (méth)acrylamido-5-salicylique;
- l'acide lactique; l'acide succinique; l'acide acétique; l'acide citrique;
- les esters de l'acide acrylique ou de l'acide méthacrylique, et notamment le (méth)acrylate d'acétoxyéthyle; l'α-hydroxy(méth)acrylate de méthyle;
- l'éthylacétoacétate de formule $CH_3$-CO-$CH_2$-$COOCH_2CH_3$, le méthylacétoacétate de formule $CH_3$-CO-$CH_2$-$COOCH_3$ et l'acétylacétone de formule $CH_3$-CO-$CH_2$-CO-$CH_3$;
- l'EDTA;
- les polyéthers de faible poids moléculaire (n compris entre 1 et 12) tels que les poly(éthylène glycol) et les poly (propylène glycol);
- la lysine et ses dérivés, notamment l' ε-N-(méth)acryloyl-L-lysine;
- la cystéine et ses dérivés, notamment la N-acétylcystéine, les disulfures de la N-acétylcystéine, la carboxyméthylcystéine;
- la cystine; la méthionine;
- les esters de l'acide lactique ou de l'acide acétique,
- la triéthanolamine;
- la cystéine et ses dérivés;
- les acides lipoïques;
- la dextrine, la cyclodextrine;
- les polymères de type polyéthylène glycol, polypropylène glycol ou polyéthylèneimine.
- les dicétones telles que la 2,4-pentanedione, la 2,4-hexafluoropentanedione, la 2,2,6,6-tétraméthyl-3, 5-heptanedione.

De préférence, le précurseur métallique selon l'invention est choisi parmi, seul ou en mélange :

- le tétraméthoxysilane, le tétraéthoxyde de silicium, de titane ou d'étain; le tétraisopropoxyde de titane, de silicium ou d'étain; le tétrabutoxyde d'étain, de titane ou de silicium;
- le méthyltriéthoxysilane, le méthyltriméthoxysilane, le mercaptopropyltriéthoxysilane, le 3-aminopropyltriéthoxysilane; l'allyltriéthoxysilane;
- le chlorure de N-triéthoxysilylpropyl-N,N,N-tri-n-butylammonium de formule $(C_4H_9)_3N^+CH_2CH_2CH_2Si(OC_2H_5)_3$, $Cl^-$
- le bromure de N-triéthoxysilylpropyl-N,N,N-tri-n-butylammonium de formule $(C_4H_9)_3N^+CH_2CH_2CH_2Si(OC_2H_5)_3$, $Br^-$
- le chlorure de N-(trimethoxysilylpropyl)-isothiouronium de formule $(NH_2)_2C{=}S^+CH_2CH_2CH_2Si(OCH_3)_3$
- le (3-glycidyloxypropyl)triméthoxysilane;
- le (3-(2-aminoéthylamino)propyl)triméthoxysilane;
- le (3-(2-(2-aminoéthylamino)éthylamino)propyl)triméthoxysilane;
- le (4-aminobutyl)triéthoxysilane;
- le (N-(6-aminohexyl)aminopropyl)triméthoxysilane;
- le (N-méthylaminopropyl)triméthoxysilane;
- l'acétoxyméthyltriéthoxysilane;
- le triéthoxysilylpropyl-3-urée;
- le triéthoxysilane;
- le (3-aminopropyl)méthyldiéthoxysilane,
- le (mercaptométhyl)méthyldiéthoxysilane,
- le (3-mercaptopropyl)méthyldiméthoxysilane,
- le bis(triéthanolamine) diisopropoxyde de titane de formule $[(HOCH_2CH_2)_2NCH_2CH_2O]_2Ti(OC_3H_7)_2$
- le méthyldiéthoxysilane, le méthyldiméthoxysilane, l'allyldiméthoxysilane;
- le bis(2,4-pentanedionate) diisopropoxyde de titane de formule :

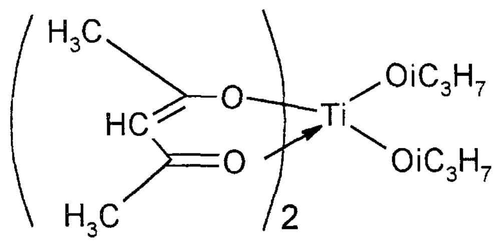

- le bis(2,2,6,6-tétraméthyl-3,5-heptanedionate) diisopropoxyde de zirconium;
- le bis(2,4-pentanedionate)titanium-O,O'-bis(oxyéthyl)aminopropyltriéthoxysilane.

Le composé métallo-organique employé dans la cadre de la présente invention est donc susceptible d'être obtenu par hydrolyse, partielle ou complète, et condensation, partielle ou complète, d'au moins un précurseur métallique tel que défini ci-dessus, selon un procédé sol-gel bien connu.

De manière générale, mais non limitative, le précurseur métallique peut initialement être solubilisé ou dispersé dans un co-solvant tel qu'une huile d'origine végétale, minérale, organique ou synthétique, telles que celles décrites ci-après, et/ou un alcool, monoalcool ou polyol, notamment de l'éthanol, tels que ceux décrits ci-après.
On procède ensuite à une réaction d'hydrolyse soit en ajoutant de l'eau, soit grâce à l'eau résiduelle, soit en ajoutant des 'générateurs' d'eau (l'eau sera alors générée in situ). On peut éventuellement introduire en outre un composé chélatant.

On obtient ainsi un sol du composé métallo-organique recherché, qui peut se présenter sous forme de particules colloïdales en suspension dans ledit co-solvant, ou sous forme de composé solubilisé dans ledit co-solvant. Les particules colloïdales sont généralement nanométriques, de préférence de l'ordre de 0,2 à 100 nanomètres, notamment 0,5-50 nm, voire 1-10 nm.
On entendra dans la suite de la présente description, par sol de composé métallo-organqiue, le mélange dudit composé métallo-organique et de son co-solvant.

De préférence, le sol de composé métallo-organique présente un extrait sec d'environ 1-95% en poids, notamment 3-90% en poids et en particulier 4-60% en poids. L'extrait sec est mesuré après chauffage du sol à 100°C, pendant 1 heure, à pression ambiante (1 atm.).

Le co-solvant susceptible d'être employé peut être choisi parmi les solvants alcooliques tels que les alcools en $C_{1-10}$, tels que le méthanol, l'éthanol, l'isopropanol, le n-propanol, le n-butanol, l'isobutanol, le t-butanol, le n-pentanol, l'hexanol; les polyols tels que le propylèneglycol, l'éthylèneglycol, le pentylèneglycol, le glycérol, le sorbitol; le Miglyol ®.
Il est également possible d'ajouter audit solvant alcoolique 0-99,9% d'eau pour obtenir un mélange hydroalcoolique.

Il est également possible d'utiliser comme co-solvant :

- les cétones comme l'acétone, la méthyl-éthyl-cétone, la méthyl-isobutyl-cétone, la diisobutylcétone, l'isophorone, la cyclohexanone;
- les éthers de glycols notamment le monométhyléther de propylèneglycol, l'acétate de monométhyléther de propylèneglycol, le monobutyléther de dipropylèneglycol;
- les aldéhydes;
- les esters et notamment les acétates comme l'acétate de butyle, de propyle, d'éthyle, d'isopropyle, d'isopentyle, de méthoxy-2-éthyle ou l'huile de Purcellin ou le myristate d'isopropyle;
- les esters d'acide minéral et d'alcool;
- les hydrocarbures linéaires ou ramifiés éventuellement aromatiques, tels que l'hexane, l'octane, l'hexadécane, l'heptane, le dodécane, le cyclohexane, l'huile de paraffine, le xylène et le toluène, les polydécènes, le polyisobutène hydrogéné tel que le parléam ;
- les éthers et les polyéthers.

On peut encore employer des huiles cosmétiquement acceptables, polaires ou apolaires, volatiles et/ou non volatiles, par exemple d'origine végétale, minérale, animale et/ou de synthèse, parmi lesquelles on peut citer, seules ou en mélange:

- les huiles hydrocarbonées d'origine animale telles que le perhydrosqualène ;
- les huiles hydrocarbonées d'origine végétale telles que les triglycérides liquides d'acides gras, par exemple les huiles de tournesol, de maïs, de soja, de courge, de pépins de raisin, de jojoba, de sésame, de noisette, d'abricot,

de macadamia, de ricin, d'avocat, d'olive, de germes de blé, d'amande douce, de calophyllum, de palme; les triglycérides des acides caprylique/caprique comme ceux vendus par la société Stearineries Dubois ou ceux vendus sous les dénominations Miglyol 810, 812 et 818 par la société Dynamit Nobel ;

- des alcools gras ayant de 10 à 32 atomes de carbone comme l'octyldodécanol, le 2-butyloctanol, le 2-hexyldécanol, le 2-undécylpentadécanol, l'alcool oléique ;
- les huiles fluorées partiellement hydrocarbonées et/ou siliconées;
- les huiles siliconées comme les polyalkyl($C_1$-$C_{20}$)siloxanes, notamment les polyalkylméthylsiloxanes et plus particulièrement les polydiméthylsiloxanes, (PDMS) volatiles ou non, linéaires ou cycliques, et notamment la cyclotétradiméthylsiloxane, la cyclopentadiméthylsiloxane ou la cyclohexadiméthylsiloxane; les silicones modifiées par des groupements aliphatiques et/ou aromatiques, éventuellement fluorés, ou par des groupements fonctionnels tels que des groupements hydroxyles, thiols et/ou amines; les huiles siliconées phénylées telles que les polyphénylméthylsiloxanes ou les phényltriméthicones.
- les huiles volatiles hydrocarbonées, telles que les isoparaffines et notamment l'isododécane et l'isohexadécane.

Le sol de composé métallo-organique peut être employé tel quel en tant que composition cosmétique.

Il est également possible d'ajouter à la composition comprenant ledit sol de composé métallo-organique, les constituants usuellement employés dans le domaine d'application envisagé.

Notamment, la composition peut comprendre une matière filmogène qui peut être choisie, notamment, parmi les résines alkydes, acryliques et/ou vinyliques, les polyuréthannes, les polyesters-polyuréthannes, les polyétherpolyuréthannes, les polymères radicalaires notamment de type acrylique, acrylique styrène et/ou vinylique, les polyesters, les celluloses et dérivés cellulosiques telles que la nitrocellulose, et les résines résultant de la condensation de formaldéhyde avec une arylsulfonamide, et leurs mélanges.

Ladite matière filmogène peut être en solution ou en dispersion dans l'eau et/ou dans un solvant organique tel que le toluène, le xylène, l'acétate d'éthyle et/ou de butyle, les cétones, les éthers de glycol, les esters et les alcools tels que l'éthanol, l'isopropanol ou le butanol, et leurs mélanges.

La composition peut également comprendre un agent plastifiant et éventuellement des agents rhéologiques. Parmi les agents plastifiants, on peut citer les citrates, les phtalates, les esters et/ou le camphre. Parmi les agents rhéologiques, on peut citer les bentonites organophiles, les dérivés de cellulose, les dérivés d'acide polyacrylique réticulé, les gommes de guar ou de caroube, ainsi que les gommes de xanthane.

Lorsque la composition se présente sous forme d'émulsion, elle peut comprendre au moins un émulsionnant classique, choisi parmi les émulsionnants amphotères, anioniques, cationiques ou non ioniques, utilisés seuls ou en mélange.

La composition peut également comprendre une phase particulaire, qui peut comprendre des pigments organiques ou minéraux, et/ou des nacres et/ou des charges habituellement utilisés dans les compositions cosmétiques. On peut notamment citer les dioxydes de titane, de zirconium ou de cérium, les oxydes de zinc, de fer ou de chrome, le bleu ferrique; le noir de carbone, et les laques de baryum, strontium, calcium, aluminium; le mica recouvert d'oxyde de titane, d'oxyde de fer, de pigment naturel ou d'oxychlorure de bismuth ainsi que le mica titane coloré et la nacre naturelle; le talc, le mica, la silice, le kaolin, les poudres de Nylon et de polyéthylène, le Téflon, l'amidon, le nitrure de bore, les microsphères telles que l'Expancel (Nobel Industrie), le polytrap (Dow Corning) et les microbilles de résine de silicone (Tospearls de Toshiba, par exemple).

La composition selon l'invention peut en outre comprendre tout additif connu de l'homme du métier comme étant susceptible d'être incorporé dans une telle composition, tels que des agents d'étalement, des épaississants, des agents mouillants, des agents dispersants, des anti-mousses, des conservateurs, des tensio-actifs, des filtres UV, des colorants, des actifs cosmétiques, des vitamines et leurs dérivés, des céramides, des oligo-éléments, des hydratants tels que la glycérine, des cires, des gommes, des huiles essentielles, de l'ADN, des parfums. Bien entendu l'homme du métier veillera à choisir ce ou ces éventuels composés complémentaires, et/ou leur quantité, de manière telle que les propriétés avantageuses apportées par l'invention ne soient pas, ou substantiellement pas, altérées par l'adjonction envisagée.

De préférence, la composition susceptible d'être employée dans le cadre de la présente invention comprend 1 à 100% en poids, notamment 1,5 à 95% en poids, en particulier 10-90% en poids, voire 12-50% en poids, de sol de composé métallo-organique.

Les compositions selon l'invention peuvent être préparée par l'homme du métier sur base de ses connaissances générales et selon l'état de la technique.

Les compositions peuvent se présenter notamment sous la forme d'une solution ou suspension aqueuse, organique ou hydroalcoolique; d'une émulsion huile-dans-eau, eau-dans-huile ou multiple, notamment une crème ou un lait; d'un gel aqueux ou huileux; d'une dispersion; d'une composition à sprayer; d'un patch.

Les matières kératiniques susceptibles d'être traitées selon l'invention peuvent être les ongles des pieds ou des mains, les cils, les sourcils, les poils et/ou les cheveux.

La composition selon l'invention peut donc se présenter sous forme de composition de maquillage telle qu'un mascara, un mascara traitant; un vernis à ongles, une base pour vernis, un soin des ongles; de composition capillaire telle qu'une laque, une lotion, une mousse de coiffage, un spray de coiffage, un stick de coiffage.
L'invention est illustrée plus en détail dans les exemples suivants.

## Exemple 1

Dans un réacteur muni d'un barreau aimanté, d'un réfrigérant, et d'une ampoule à brome, on introduit de l'éthanol et de l'eau à pH1. Le mélange est placé sous agitation magnétique pendant 15 minutes à 30°C. On y ajoute goutte à goutte 10,4 g (0,05 mole) de tétraéthoxysilane sous agitation soutenue pendant 48 heures. Le mélange réactionnel est enfin coulé dans une fiole et placé à 30°C.
La composition (ou le sol) ainsi obtenue peut être conservée telle quelle, en vue de son incorporation ultérieure dans une composition cosmétique.

## Exemple 2

Dans un réacteur muni d'un barreau aimanté, d'un réfrigérant, et d'une ampoule à brome, on introduit de l'éthanol et de l'eau à pH1. Le mélange est placé sous agitation magnétique pendant 15 minutes à 30°C. On y ajoute goutte à goutte 11 g (0,05 mole) d'aminopropyltriéthoxysilane, et l'on maintient une vive agitation pendant 48 heures. Le mélange réactionnel est enfin coulé dans une fiole et placé à 30°C.
La composition ainsi obtenue peut être conservée telle quelle, en vue de son incorporation ultérieure dans une composition cosmétique.

## Exemple 3

Dans un réacteur muni d'un barreau aimanté, d'un réfrigérant, et d'une ampoule à brome, on introduit de l'éthanol et de l'eau à pH1. Le mélange est placé sous agitation magnétique pendant 15 minutes à 30°C. On y ajoute goutte à goutte 8,9 g (0,05 mole) de méthyltriéthoxysilane et l'on maintient une vive agitation pendant 48 heures. Le mélange réactionnel est enfin coulé dans une fiole et placé à 30°C.
La composition ainsi obtenue peut être conservée telle quelle, en vue de son incorporation ultérieure dans une composition cosmétique.

## Exemple 4

On effectue une caractérisation dynamique en flexion sur des ongles traités par les compositions des exemples 2 et 3 ci-dessus et par une composition durcissante comparative du commerce "Durcilong" de Gemey.
La caractérisation dynamique en flexion permet notamment de déterminer les propriétés viscoélastiques d'un matériau en terme d'élasticité et de viscosité.
Elle consiste à imposer à un échantillon une sollicitation sinusoïdale (déplacement) répétée de faible amplitude $\Delta l$. L'échantillon réagit à la sollicitation en opposant une force sinusoïdale d'amplitude $\Delta F$, déphasée par rapport à la sollicitation d'un angle $\delta$.
On peut caractériser le caractère viscoélastique du matériau par le module de raideur K (N/m) qui correspond à la rigidité globale de l'échantillon telle qu'on peut l'appréhender en tirant ou en écrasant l'échantillon; et par l'angle de perte $\delta$ (en degré) qui caractérise la viscosité intrinsèque. Plus l'angle de perte est important, plus la viscosité du matériau est importante et plus la réponse du matériau est en retard sur la sollicitation.

$$K(N/m) = \frac{\Delta F}{\Delta l}$$

Des échantillons d'ongles humains naturels sont découpés aux ciseaux en forme de rectangle de 4 mm x 5 mm.
Avant toute mesure, ils sont laissés pendant 48 heures dans une boite à gants à une température de 25°C et sous une humidité relative de 45%.
On applique sur les échantillons 3 gouttes des compositions préparées aux exemples 2 et 3, à l'aide d'une micropipette.
On laisse alors les échantillons traités pendant 24 heures en boîte à gants à une température de 25°C et sous une humidité relative de 45%, pour que le solvant s'évapore complètement.
Pour chacun des échantillons, on détermine avant et après traitement, la valeur du module de raideur K et

de l'angle de perte δ.
On utilise un viscoélasticimètre METRAVIB VA 2000, qui permet de réaliser des mesures directement en boîte à gants (T = 25°C ; H.R. = 45 %).
L'échantillon est serré dans un mors de fixation par le "côté racine" du bout d'ongle. La tête de mesure dynamique du viscoélasticimètre vient solliciter de façon répétée l'extrémité libre (bord opposé) de l'ongle par l'intermédiaire d'un couteau. A l'ongle est appliqué une déformation (en flexion) constante, sur laquelle est superposée une sollicitation sinusoïdale : la flexion ondulée. L'ongle reste globalement toujours fléchi dans le même sens.
Les conditions sont les suivantes :

- déplacement statique : $d_{stat}$ = - 300 µm environ, ce qui correspond à une force statique F de - 0,6 N
- amplitude du déplacement dynamique : $d_{dyn}$ = ± 30 µm environ
- fréquence de sollicitation sinusoïdale : *f* = 10 Hz

On détermine les valeurs de ΔF, donc de K, et de δ, avant traitement, après 1 jour et après 7 jours de traitement. On en déduit les valeurs de ΔK et de Δδ, après 1 et 7 jours.

$$\Delta K\ (\%) = \frac{K_{après} - K_{avant}}{K_{avant}} \times 100 \qquad \Delta\delta(\%) = \frac{\delta_{après} - \delta_{avant}}{\delta_{avant}} \times 100$$

On obtient les résultats suivants :

| | K avant traitement (N/m) | K après 1 jour (N/m) | K après 7 jours (N/m) | ΔK (1 jour) | ΔK (7 jours) |
|---|---|---|---|---|---|
| Exemple 2 (extrait sec 10,2%) | 6830 | 7770 | 8090 | 13,76 | 18,45 |
| Exemple 3 (extrait sec 5,9%) | 6930 | 7490 | 7770 | 8,08 | 12,12 |
| Durcilong (extrait sec : 13%) | 8770 | 9910 | 10300 | 13,00 | 17,44 |

| | δ avant traitement | δ après 1 jour | δ après 7 jours | Δδ (1 jour) | Δδ (7 jours) |
|---|---|---|---|---|---|
| Exemple 2 (extrait sec 10,2%) | 2,9 | 4,2 | 4,0 | 44,83 | 37,93 |
| Exemple 3 (extrait sec : 5,9%) | 2,8 | 3,2 | 3,4 | 14,29 | 21,43 |
| Durcilong (extrait sec : 13%) | 3,3 | 6,5 | 6,1 | 96,97 | 84,85 |

On constate donc que les composés des exemples 2 et 3 permettent d'obtenir des résultats comparables, en terme de rigidification, à ceux obtenus avec un produit renforçateur des ongles du commerce, pour des taux d'extrait sec plus faible.

<u>**Exemple 5**</u>

On effectue des mesures de dureté Vickers sur des ongles traités par les compositions de l'exemple 2 ci-dessus.
La mesure est effectuée de la manière suivante :

Des prélèvements d'ongles sont effectués sur plusieurs donneurs. Ils sont coupés sous forme de petits rectangles de largeur 2 à 3 mm dans la partie longitudinale médiane et de longueur de 3 à 4 mm environ.
Trois fragments sont collés sur un support rectangulaire en inox à l'aide d'une colle de type « cyanolite ». Il est préparé 3 plots par produit soit 9 fragments.
Les échantillons sont ensuite conditionnés à une humidité relative de 75% et à une température de 30°C, pendant

48 heures.

On effectue une première mesure sur les échantillons non traités.

Puis, on applique 0,5 µl par $mm^2$ de la composition à tester sur chaque fragments d'ongles. Les fragments sont ensuite conditionnés à une humidité relative de 75% et à une température de 30°C pendant 48 heures.

On détermine le coefficient de dureté Vickers (HV) à l'aide d'un microduromètre M 400 G2 LECO® , avec les conditions suivantes :

- charge d'application : 50 gf
- temps d'application : 15 sec
- vitesse d'application : 60 µm/sec

On obtient les résultats suivants:

| | Dureté Vickers (HV) |
|---|---|
| ongles non traités | 16 |
| ongles traités avec la composition de l'exemple 2 | 18 |

On observe donc une augmentation d'environ 12,5% de la dureté des ongles traités par la composition selon l'invention.

**<u>Exemple 6</u>**

On prépare une composition comprenant, en poids :

- épaississant 2%
- colorant 0,2%
- conservateur, parfum qs
- composition de l'exemple 1 qsp 100%

On obtient une composition pouvant s'appliquer sur les ongles.

## Revendications

**1.** Utilisation d'une composition comprenant au moins un composé métallo-organique susceptible d'être obtenu par hydrolyse, partielle ou complète, et condensation, partielle ou complète, d'au moins un précurseur métallique choisi parmi, seul ou en mélange :

(1) les alcoxydes métalliques répondant à l'une des formules (Ia), (Ib), (Ic) ou (Id) suivantes :

$$M\text{-}(OR_1)_n \qquad \text{(I a)}$$

$$R\text{-}M\text{-}(OR_1)_{n-1} \qquad \text{(I b)}$$

$$(R_1O)_{n-1}\text{-}M\text{-}R''\text{-}M'\text{-}(OR_1)_{n-1} \qquad \text{(I c)}$$

$$RR'\text{-}M\text{-}(OR_1)_{n-2} \qquad \text{(I d)}$$

dans lesquelles :

- M et M', indépendamment l'un de l'autre, désignent un atome de métal choisi parmi les métaux de transition

des groupes Ib à VIIb, du groupe VIII ou du groupe des lanthanides de la classification périodique, l'aluminium, le silicium, le bore, l'étain et le magnésium, les métaux alcalins et alcalino-terreux;

- n désigne la valence du métal;
- $R_1$, identiques ou différents, désignent un radical hydrocarboné, linéaire ou ramifié, saturé ou insaturé, ayant 1 à 30 atomes de carbone, de préférence 1 à 6 atomes de carbone, éventuellement interrompu ou substitué par 1-20 hétéroatomes choisis parmi O, N, S et/ou P;
- R et R', indépendamment l'un de l'autre, désignent un atome d'hydrogène ou un radical hydrocarboné, linéaire, ramifié ou cyclique, saturé ou insaturé, en C1-30, de préférence C2-20, éventuellement substitué ou interrompu par 1-20 hétéroatomes choisis parmi O, N, S et/ou P; et/ou substitué par un groupement choisi dans la liste ci-après;
  lesdits R et/ou R' pouvant comporter en outre un ou plusieurs groupes cosmétiquement actifs tels que définis ci-après;
- R" désigne -O-, -$NR^2$-, -S- ou un radical divalent hydrocarboné, linéaire, cyclique ou ramifié, saturé ou insaturé, en C1-30, de préférence C2-20, éventuellement substitué par un groupement choisi dans la liste ci-après; et/ou éventuellement interrompu ou substitué par 1-20 hétéroatomes choisis parmi O, N, P et/ou S, pouvant comporter en outre un ou plusieurs groupes cosmétiquement actifs tels que définis ci-après, avec $R^2$ représentant un radical hydrocarboné, linéaire, cyclique ou ramifié, saturé ou insaturé, en C1-30, de préférence C2-20;

(2) les complexes répondant à l'une des formules (IIa), (IIb), (IIc) ou (IId) suivantes :

$$M\text{-}(OR_1)_{n-x}\,(X)_x \qquad \text{(II a)}$$

$$R\text{-}M\,(OR_1)_{n-1-x}(X)_x \qquad \text{(II b)}$$

$$(X)_x(R_1O)_{n-1-x}\,M\text{-}R''\text{-}M'\text{-}(OR_1)_{n-1-x}\,(X)_x \qquad \text{(II c)}$$

$$RR'\text{-}M\text{-}(OR_1)_{n-x-2}(X)_x \qquad \text{(II d)}$$

dans lesquelles :

- M, M', n, R, R', R" et $R_1$ ont la même signification que ci-dessus;
- X désigne un ligand comportant un atome d'azote, de phosphore, de soufre et/ou d'oxygène, et portant éventuellement un groupe cosmétiquement actif tel que défini ci-après;
- x est le nombre d'atomes pouvant se lier à l'atome central métallique;

(3) les halogénures métalliques répondant à l'une des formules (IIIa), (IIIb), (IIIc) ou (IIId) suivantes :

$$M\text{-}(Z)_n \qquad \text{(III a)}$$

$$R\text{-}M\text{-}(Z)_{n-1} \qquad \text{(III b)}$$

$$(Z)_{n-1}\text{-}M\text{-}R''\text{-}M'\text{-}(Z)_{n-1} \qquad \text{(III c)}$$

$$RR'\text{-}M\text{-}(Z)_{n-2} \qquad \text{(III d)}$$

dans lesquelles :

- M, M', n, R, R' et R" ont la même signification que ci-dessus;

- Z représente, indépendamment l'un de l'autre, un atome d'halogène (chlore, iode, brome ou fluor);

(4) les complexes répondant à l'une des formules (IVa), (IVb), (IVc) ou (IVd) suivantes :

$$M\text{-}(Z)_{n-x}\,(X)_x \qquad \text{(IVa)}$$

$$R\text{-}M\,(Z)_{n-1-x}\,(X)_x \qquad \text{(IVb)}$$

$$(X)_x\,(Z)_{n-1-x}\,M\text{-}R''\text{-}M'\text{-}(Z)_{n-1-x}\,(X)_x \qquad \text{(IVc)}$$

$$RR'\text{-}M\text{-}(Z)_{n-x-2}\,(X)_x \qquad \text{(IVd)}$$

dans lesquelles :

- M, M', n, R, R', R", X, x et Z ont la même signification que ci-dessus;

pour protéger et/ou renforcer les matières kératiniques.

2. Utilisation selon la revendication 1, pour améliorer de manière rapide et durable, la rigidité et/ou la cohésion desdites matières kératiniques.

3. Utilisation selon l'une des revendications précédentes, dans laquelle lesdites matières kératiniques sont choisies parmi les ongles des pieds ou des mains, les cils, les sourcils, les poils et les cheveux.

4. Utilisation selon l'une des revendications précédentes, pour diminuer la fragilisation des ongles, notamment des ongles affaiblis, en particulier des ongles striés, fendus, mous, souples, et/ou ayant tendance à se dédoubler; pour obtenir des ongles plus durs, plus résistants, moins cassants; pour obtenir des ongles qui ne se dédoublent plus et/ou qui ne se fendillent plus.

5. Utilisation selon l'une des revendications 1 à 3, pour rigidifier les cheveux, notamment les cheveux mous, et en améliorer ainsi le coiffage.

6. Utilisation selon l'une des revendications précédentes, dans laquelle le précurseur métallique est choisi par les composés répondant aux formules (Ia), (Ib) et (IIa).

7. Utilisation selon l'une des revendications précédentes, dans laquelle l'atome de métal M est choisi parmi le titane, le zirconium, l'aluminium, le fer, l'étain et le silicium, et plus particulièrement parmi le titane et le silicium.

8. Utilisation selon l'une des revendications précédentes, dans laquelle:

- $R_1$ désigne, identique ou différent, un radical hydrocarboné, linéaire ou ramifié, saturé, ayant 1 à 30 atomes de carbone, de préférence 1 à 6 atomes de carbone, et plus particulièrement un radical méthyl, éthyl, propyl, n-butyl, isobutyl ou t-butyl; et/ou
- R et R' désignent, indépendamment l'un de l'autre, un radical hydrocarboné, linéaire ou ramifié, saturé, en C1-20, notamment C1-6; ou un radical hydrocarboné, linéaire ou ramifié, saturé, en C1-20, notamment C1-6, substitué par un ou plusieurs atomes d'halogène (notamment perfluoré), ou par un groupement-$NH_2$, -CO-$NH_2$, -SH, -$CO_2H$,-COR, -OH, -N=C=O,-NH-CO-$NH_2$, -$N^+R_3$ et notamment -$N^+Bu_3$, -$S^+$=$C(NH_2)_2$; benzènesulfonate;

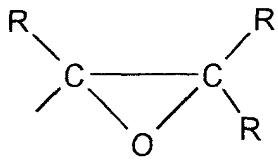 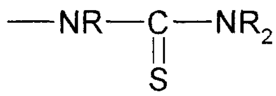 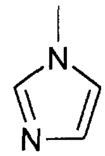 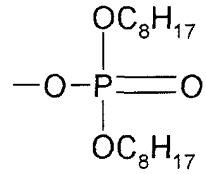

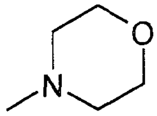 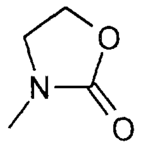 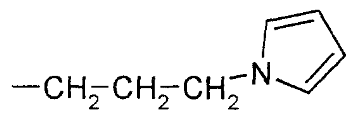

dans lesquels les différents radicaux R, identiques ou différents, désignent un atome d'hydrogène ou un radical hydrocarboné, linéaire, ramifié ou cyclique, saturé ou insaturé, en C1-30, de préférence C2-20; et/ou

- R" désigne -O-, -NH- ou un radical divalent hydrocarboné, linéaire ou ramifié, saturé, en C1-30, de préférence C2-20, éventuellement interrompu par au moins un hétéroatome choisi parmi O, N, P et/ou S; et/ou
- X est choisi parmi les acides carboxyliques, les acides sulfoniques, les acides phosphoniques, les acides phosphoriques, les acides sulfuriques, les cétones, les β-dicétones, les esters, les β-cétoesters, les amines, les β-cétoamines, les acides aminés de préférence α- ou β-hydroxylés et leurs dérivés, les α- ou β-hydroxyacides, les éthers et polyéthers, les imines, les amides éventuellement hydroxylées, les composés azoïques, les thiols, les ureidos, les sulfoxydes de thioéther, les sulfones de thioéther, les thioéthers éventuellement cycliques, les di(thioéthers), les monoalcools ou les polyols, la dextrine et ses dérivés, les thiazolidines; les polymères hydrocarbonés comportant éventuellement des hétéroatomes choisis parmi N, O, S et/ou P, susceptibles d'être obtenus par polymérisation radicalaire, par condensation ou par polymérisation 'vivante' contrôlée, ayant un poids moléculaire (en masse) compris entre 90 et 10000, notamment de 100 à 1000, voire de 150 à 500; et leurs dérivés.

**9.** Utilisation selon l'une des revendications précédentes, dans laquelle le précurseur métallique est choisi parmi, seul ou en mélange :

- le tétraméthoxysilane, le tétraéthoxyde de silicium, de titane ou d'étain; le tétraisopropoxyde de titane, de silicium ou d'étain; le tétrabutoxyde d'étain, de titane ou de silicium;
- le méthyltriéthoxysilane, le méthyltriméthoxysilane, le mercaptopropyltriéthoxysilane, le 3-aminopropyltriéthoxysilane; l'allyltriéthoxysilane;
- le chlorure de N-triéthoxysilylpropyl-N,N,N-tri-n-butylammonium de formule $(C_4H_9)_3N^+CH_2CH_2CH_2Si(OC_2H_5)_3$, $Cl^-$
- le bromure de N-triéthoxysilylpropyl-N,N,N-tri-n-butylammonium de formule $(C_4H_9)_3N^+CH_2CH_2CH_2Si(OC_2H_5)_3$, $Br^-$
- le chlorure de N-(trimethoxysilylpropyl)-isothiouronium de formule $(NH_2)_2C{=}S^+CH_2CH_2CH_2Si(OCH_3)_3$
- le (3-glycidyloxypropyl)triméthoxysilane;
- le (3-(2-aminoéthylamino)propyl)triméthoxysilane;
- le (3-(2-(2-aminoéthylamino)éthylamino)propyl)triméthoxysilane;
- le (4-aminobutyl)triéthoxysilane;
- le (N-(6-aminohexyl)aminopropyl)triméthoxysilane;
- le (N-méthylaminopropyl)triméthoxysilane;
- l'acétoxyméthyltriéthoxysilane;
- le triéthoxysilylpropyl-3-urée;
- le triéthoxysilane;
- le (3-aminopropyl)méthyldiéthoxysilane,
- le (mercaptométhyl)méthyldiéthoxysilane,
- le (3-mercaptopropyl)méthyldiméthoxysilane,
- le bis(triéthanolamine) diisopropoxyde de titane de formule $[(HOCH_2CH_2)_2NCH_2CH_2O]_2Ti(OC_3H_7)_2$
- le méthyldiéthoxysilane, le méthyldiméthoxysilane, l'allyldiméthoxysilane;

- le bis(2,4-pentanedionate) diisopropoxyde de titane de formule :

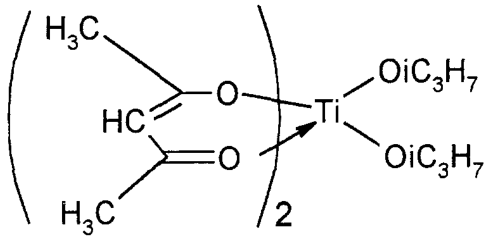

- le bis(2,2,6,6-tétraméthyl-3,5-heptanedionate) diisopropoxyde de zirconium;
- le bis(2,4-pentanedionate)titanium-O,O'-bis(oxyéthyl)aminopropyltriéthoxysilane.

**10.** Utilisation selon l'une des revendications précédentes, dans laquelle la composition comprend 1 à 100% en poids, notamment 1,5 à 95% en poids, en particulier 10-90% en poids, voire 12-50% en poids, de sol de composé métallo-organique.

**11.** Utilisation selon l'une des revendications précédentes, dans laquelle la composition se présente sous forme de composition de maquillage telle qu'un mascara, un mascara traitant; un vernis à ongles, une base pour vernis, un soin des ongles; de composition capillaire telle qu'une laque, une lotion, une mousse de coiffage, un spray de coiffage, un stick de coiffage.

**12.** Procédé de traitement des matières kératiniques, notamment pour les protéger et/ou les renforcer, consistant à appliquer sur lesdites matières une composition comprenant au moins un composé métallo-organique susceptible d'être obtenu par hydrolyse, partielle ou complète, et condensation, partielle ou complète, d'au moins un précurseur métallique tel que défini dans la revendication 1.

Office européen des brevets

# RAPPORT DE RECHERCHE EUROPEENNE

Numéro de la demande
EP 01 40 1581

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X | WO 98 44906 A (L'OREAL) 15 octobre 1998 (1998-10-15) * le document en entier * --- | 1-12 | A61K7/043 |
| P,X | WO 00 53153 A (L'OREAL) 14 septembre 2000 (2000-09-14) * le document en entier * ----- | 1-12 | |

| DOMAINES TECHNIQUES RECHERCHES (Int.Cl.7) |
|---|
| A61K |

Le présent rapport a été établi pour toutes les revendications

| Lieu de la recherche | Date d'achèvement de la recherche | Examinateur |
|---|---|---|
| LA HAYE | 25 octobre 2001 | Willekens, G |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

## ANNEXE AU RAPPORT DE RECHERCHE EUROPEENNE RELATIF A LA DEMANDE DE BREVET EUROPEEN NO.

EP 01 40 1581

La présente annexe indique les membres de la famille de brevets relatifs aux documents brevets cités dans le rapport de recherche européenne visé ci–dessus.
Lesdits members sont contenus au fichier informatique de l'Office européen des brevets à la date du
Les renseignements fournis sont donnés à titre indicatif et n'engagent pas la responsabilité de l'Office européen des brevets.

25-10-2001

| Document brevet cité au rapport de recherche | | Date de publication | Membre(s) de la famille de brevet(s) | | | Date de publication |
|---|---|---|---|---|---|---|
| WO 9844906 | A | 15-10-1998 | AU | 717355 | B2 | 23-03-2000 |
| | | | AU | 7055398 | A | 30-10-1998 |
| | | | BR | 9808454 | A | 23-05-2000 |
| | | | CN | 1251519 | T | 26-04-2000 |
| | | | EP | 0971685 | A1 | 19-01-2000 |
| | | | WO | 9844906 | A1 | 15-10-1998 |
| | | | HU | 0002337 | A2 | 28-12-2000 |
| | | | JP | 2000510167 | T | 08-08-2000 |
| | | | PL | 336001 | A1 | 05-06-2000 |
| WO 0053153 | A | 14-09-2000 | FR | 2790664 | A1 | 15-09-2000 |
| | | | AU | 3295500 | A | 28-09-2000 |
| | | | WO | 0053153 | A1 | 14-09-2000 |

EPO FORM P0460

Pour tout renseignement concernant cette annexe : voir Journal Officiel de l'Office européen des brevets, No.12/82